(19) Europäisches Patentamt
European Patent Office
Office européen des brevets

(11) Veröffentlichungsnummer : **0 105 210**
**B1**

(12) # EUROPÄISCHE PATENTSCHRIFT

(45) Veröffentlichungstag der Patentschrift : **04.03.87**

(21) Anmeldenummer : **83108697.0**

(22) Anmeldetag : **03.09.83**

(51) Int. Cl.$^4$ : **C 07 D217/26**, C 07 D401/12, C 07 D413/12, C 07 D471/12, C 07 D491/056, A 61 K 31/47, A 61 K 31/395

(54) **Isochinolinderivate, Verfahren zu ihrer Herstellung, pharmazeutische Präparate auf Basis dieser Verbindungen und ihre Verwendung.**

(30) Priorität : **09.09.82 DE 3233424**

(43) Veröffentlichungstag der Anmeldung : **11.04.84 Patentblatt 84/15**

(45) Bekanntmachung des Hinweises auf die Patenterteilung : **04.03.87 Patentblatt 87/10**

(84) Benannte Vertragsstaaten : **AT BE CH DE FR GB IT LI LU NL SE**

(56) Entgegenhaltungen : **EP-A- 0 005 231**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Konz, Elmar, Dr.**
**Brüningstrasse 9**
**D-6233 Kelkheim (Taunus) (DE)**
Erfinder : **Kaiser, Joachim, Dr.**
**Fichtestrasse 12**
**D-6000 Frankfurt am Main (DE)**

**EP 0 105 210 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Isochinolinderivate mit Substituenten in 4-Stellung sind häufig in der Literatur beschrieben, so z. B. Nippon Kagaku Zasshi *92,* (1971) 1, 80-82 oder Tetrahedron Letters *1971,* 47, 4503-4506, doch wurden keine biologischen Wirkungen angegeben. In der belgischen Patentschrift 875 797 werden unter anderem 4-(4,5-Dihydro-2-imidazolylamino)isochinolinderivate mit antihypertensiven Eigenschaften beschrieben und in der niederländischen Patentanmeldung 7 010 358 sind Isochinolinderivate aufgeführt, die Papaverinähnliche antispasmodische Aktivitäten besitzen. 4-Amino-6,7-dimethoxyisochinolin wird in der Zeitschrift J. of Pharmaceutical Sciences *63,* 149 (1974) als blutdrucksenkende Substanz beschrieben.

Aus der EP-A 5 231 sind Isochinolinderivate bekannt, die sich von den erfindungsgemäßen durch 3-Halogensubstitution des Isochinolins unterscheiden. Sie weisen ebenfalls antiarrhythmische Wirkung auf.

Aufgabe der Erfindung war es, eine deutliche Verbesserung der bekannten Verbindungen zu erzielen.

So wurde überraschenderweise gefunden, daß basisch substituierte Isochinolin-4-carbonsäurederivate antiarrhythmische Wirkung besitzen.

Die vorliegende Erfindung betrifft deshalb Isochinolin-4-carbonsäurederivate der Formel I

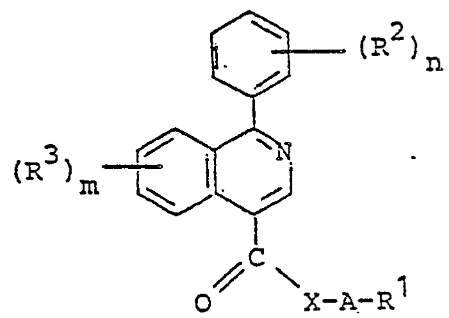

(I)

sowie deren physiologisch verträgliche Salze, Verfahren zur Herstellung dieser Verbindungen, ihre Verwendung als Arzneimittel sowie pharmazeutische Präparate, die diese Verbindungen enthalten.

In der Formel I bedeuten :

m und n eins oder zwei,

X Sauerstoff oder Stickstoff, der mit Wasserstoff oder $C_1$-$C_6$-Alkyl substituiert ist,

A eine Bindung oder eine geradkettige oder verzweigte $C_1$-$C_8$-Alkylenkette,

$R^1$ entweder eine Aminogruppe der Formel

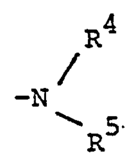

worin $R^4$ und $R^5$ gleich oder verschieden sind und Wasserstoff oder geradkettige oder verzweigte $C_1$-$C_8$-Alkylreste bedeuten oder

$R^1$ einen 3- bis 8-gliedrigen ein Stickstoffatom enthaltenden, entweder über ein Kohlenstoffatom oder ein Stickstoffatom mit A verbundenen Ring, worin das Stickstoffatom gegebenenfalls mit Wasserstoff oder $C_1$-$C_8$-Alkyl substituiert ist, und worin eine —$CH_2$-Gruppe durch Sauerstoff, Schwefel oder die —NH— oder N—$C_1$-$C_8$-Alkylgruppe ersetzt sein kann, und mit der Maßgabe, daß die Heteroatome in der Seitenkette (—X—A—$R^1$) durch mindestens 2 Kohlenstoffatome getrennt sind,

$R^2$ Wasserstoff, Halogen, Hydroxy, Nitro, Amino, $C_1$-$C_6$-Alkyl- oder $C_1$-$C_6$-Alkoxyreste und

$R^3$ Wasserstoff, Halogen, Hydroxy, Nitro, Amino, $C_1$-$C_6$-Alkyl- oder $C_1$-$C_6$-Alkoxyreste, Benzyloxy-, Methylendioxy- oder Äthylendioxy-Gruppe.

Insbesondere beinhaltet die Erfindung Verbindungen, worin m und n eins oder zwei, X Sauerstoff oder Stickstoff, der mit Wasserstoff oder $C_1$-$C_4$-Alkyl substituiert ist, A eine Bindung oder eine geradkettige oder verzweigte $C_1$-$C_6$-Alkylenkette bedeuten, $R^1$ entweder eine Aminogruppe der Formel

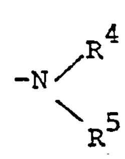

bedeutet, wobei $R^4$ und $R^5$ gleich oder verschieden sind und Wasserstoff oder geradkettige oder verzweigte $C_1$-$C_6$-Alkylreste bedeuten oder $R_1$ einen 3- bis 6-gliedrigen ein Stickstoffatom enthaltenden Ring bedeutet, wobei das Stickstoffatom gegebenenfalls mit Wasserstoff oder $C_1$-$C_6$-Alkyl substituiert ist, und wobei eine —$CH_2$-Gruppe durch Sauerstoff, Schwefel oder die —NH— oder —N-$C_1$-$C_6$-Alkylgruppe ersetzt sein kann, und mit der Maßgabe, daß die Heteroatome in der Seitenkette mindestens durch 2 Kohlenstoffatome getrennt sind, $R^2$ Wasserstoff, Halogen, Hydroxy, Nitro, Amino, $C_1$-$C_4$-Alkyl- oder $C_1$-$C_4$-Alkoxyreste bedeutet und $R^3$ Wasserstoff, Halogen, Hydroxy, Nitro, Amino, $C_1$-$C_4$-Alkyl- oder $C_1$-$C_4$-Alkoxyreste darstellt.

Besonders bevorzugt sind Verbindungen, worin m und n eins oder zwei, X Sauerstoff oder Stickstoff, der mit Wasserstoff, Methyl- oder Äthylrest substituiert ist, A eine Bindung oder eine geradkettige oder verzweigte $C_1$-$C_4$-Alkylenkette, insbesondere die Methylen-, Äthylen-, Propylen-, iso-Propylen-, Butylen- und iso-Butylen-Kette bedeuten, $R^1$ entweder eine Aminogruppe der Formel

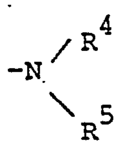

bedeutet, wobei $R^4$ und $R^5$ gleich oder verschieden sind und Wasserstoff oder geradkettige oder verzweigte $C_1$-$C_4$-Alkylreste bedeuten, insbesondere den Methyl-, Äthyl-, Propyl- und Butylrest oder $R^1$ einen 3- bis 6-gliedrigen ein Stickstoffatom enthaltenden Ring bedeutet, insbesondere den Aziridino-, Pyrrolidino- und Piperidinoring, wobei der Stickstoff gegebenenfalls mit Wasserstoff, Methyl, Äthyl, Propyl-, iso-Propyl oder Butyl substituiert ist, oder den Piperazinoring, der mit den genannten Alkylresten substituiert sein kann und mit der Maßgabe, daß die Heteroatome in der Seitenkette mindestens durch 2 Kohlenstoffatome getrennt sind, $R^2$ Wasserstoff, Fluor, Chlor, Brom, Hydroxy, Amino, Methyl, Äthyl, Methoxy oder Äthoxy, bevorzugt in ortho oder/und para-Position bedeutet und $R^3$ Wasserstoff, Fluor, Chlor, Brom, Hydroxy, Amino, Methyl, Äthyl, Methoxy oder Äthoxy, bevorzugt in 6- oder/und 7-Position darstellt.

Von ganz besonderem Interesse sind Verbindungen, worin n eins, X Sauerstoff oder Stickstoff, der mit Wasserstoff substituiert ist, A eine geradkettige $C_2$ bis $C_3$-Alkylengruppe, $R^1$ eine Aminogruppe der Formel

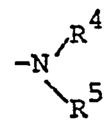

worin $R^4$ und $R^5$ gleich sind und $C_1$-$C_3$-Alkyl darstellen, $R^2$ Wasserstoff oder $C_1$-$C_3$-Alkyl und $R^3$ Wasserstoff bedeuten.

Das Verfahren zur Herstellung der Verbindungen der Formel I ist dadurch gekennzeichnet, daß man Verbindungen der Formel II

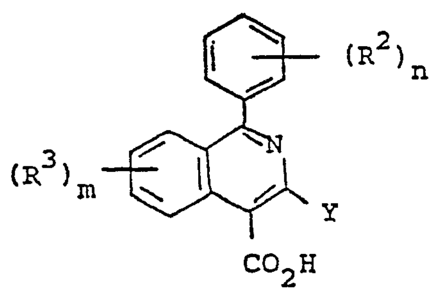

(II)

worin m, n, $R^2$ und $R^3$ die zu Formel I genannte Bedeutung haben und Y Wasserstoff oder Halogen bedeutet, mit einem Amin oder Alkohol der Formel HX—A—$R^1$, worin X, A, und $R^1$ die obengenannte Bedeutung haben, umsetzt, eine erhaltene Verbindung, worin Y Halogen bedeutet, enthalogeniert, und die Reaktionsprodukte gegebenenfalls in die physiologisch verträglichen Säureadditionssalze überführt.

Die Umsetzung der Carbonsäuren der Formel II mit einem Amin oder Alkohol der Formel HX—A—$R^1$ erfolgt nach an sich bekannten Methoden, zweckmäßig in Gegenwart eines Kondensationsmittels wie z. B. Thionylchlorid. Der Substituent Y ist vorzugsweise Wasserstoff oder Chlor.

Die Verbindungen II sind entweder in der Literatur beschrieben oder können analog den in der Literatur beschriebenen Methoden hergestellt werden. Besonders geeignet sind folgende Methoden :

a) Nippon Nogei Kagaku Kaishi *1973*, 47, (1), 23-36

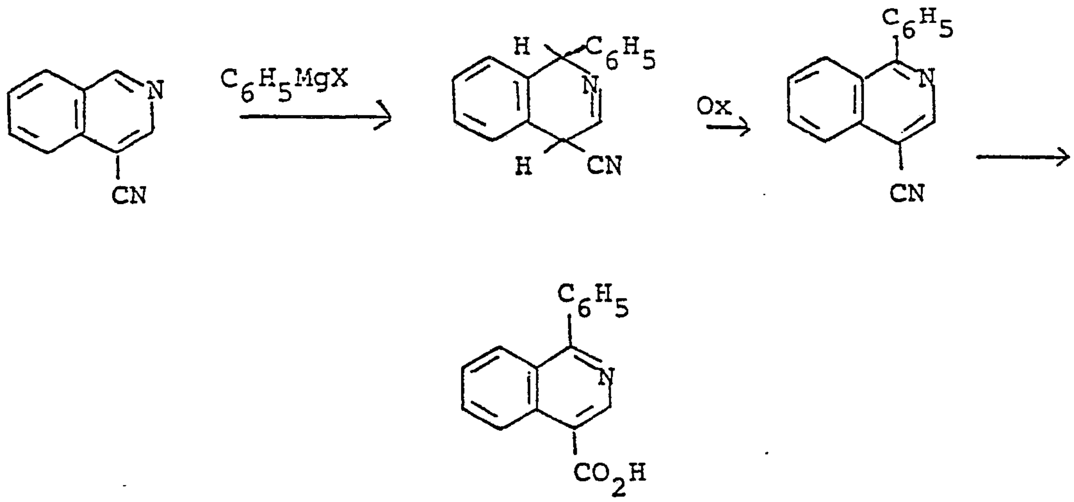


b) Bl. *1966,* 5, 1763-1765 :

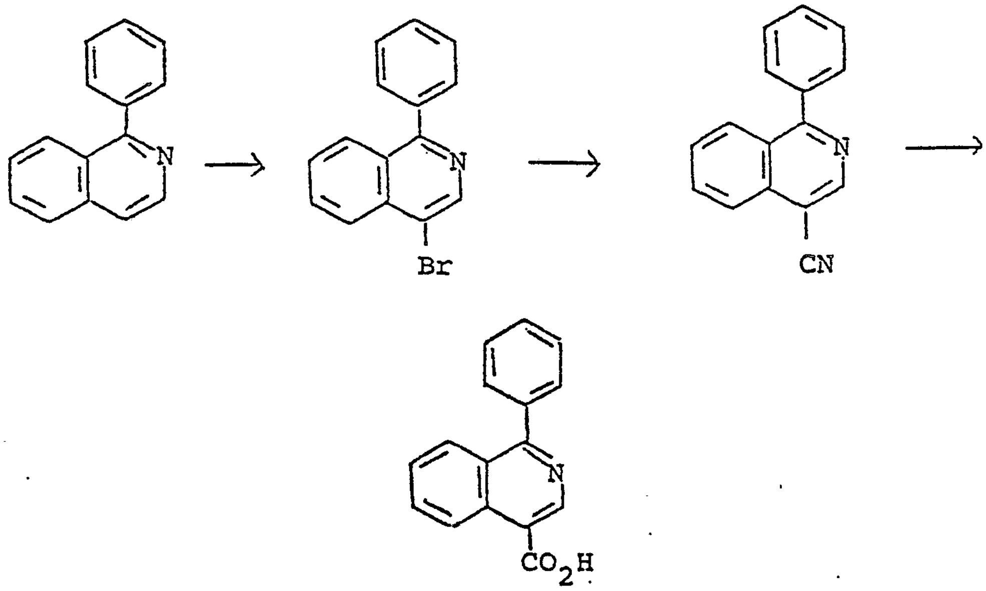

c) Eine andere Darstellung der Verbindungen II geht aus von 3-Chlor-1-phenylisochinolin-4-carbonsäuren, die in der DE-OS 28 18 423 beschrieben sind :

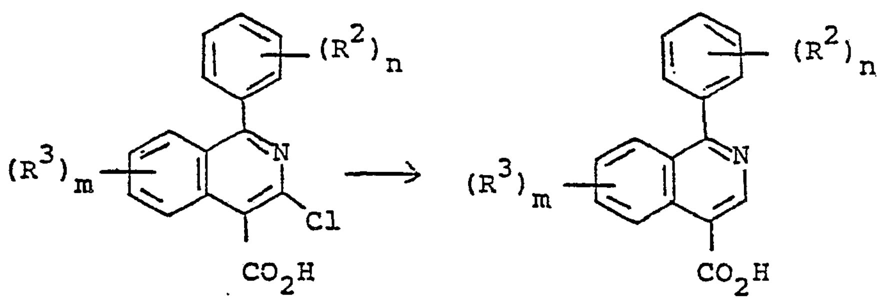

Die Enthalogenierung gelingt am besten durch katalytische Hydrierung mit z. B. Palladium auf Tierkohle oder Platinoxid in Methanol, oder anderen für Hydrierung gängigen Lösungsmitteln bei Temperaturen zwischen 20-100 °C und 1-100 atm. Wasserstoffdruck in Gegenwart einer Base wie z. B. Ammoniak oder Natriumhydroxid.

Vertauscht man die beiden Reaktionsschritte Enthalogenierung und Amidbildung ausgehend z. B. von 3-Chlor-1-phenyl-isochinolin-4-carbonsäuren, so ergibt sich folgende Synthesesequenz für die Herstellung der Verbindung I :

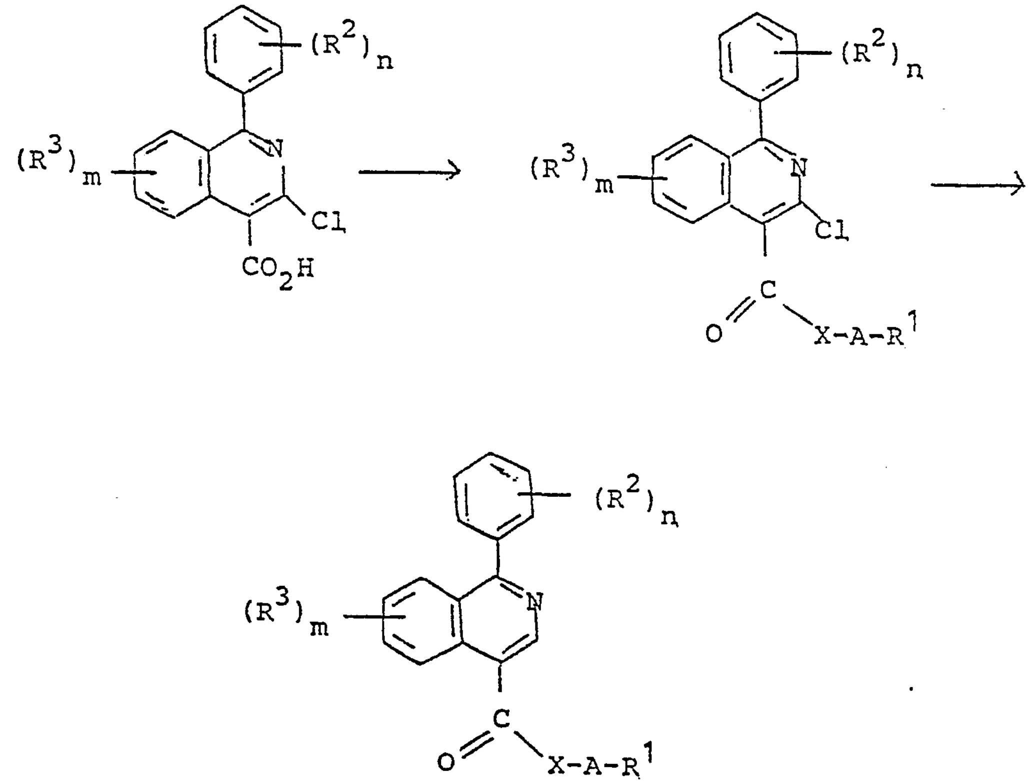

Die Enthalogenierung wird zweckmäßigerweise wie vorstehend angegeben durchgeführt. Nach diesen Verfahren können folgende Verbindungen dargestellt werden :

N-(2-(Diethylamino)ethyl)-1-(2,4-dimethylphenyl)isochinolin-4-carbonsäureamid
1-(2,4-Dimethylphenyl)isochinolin-4-carbonsäure, (2-(Diethylamino)ethyl)ester
N-(2-(Diethylamino)ethyl)-1-(2,3-dimethylphenyl)isochinolin-4-carbonsäureamid
N-(2-(Diethylamino)ethyl)-6-methoxy-1-(2-methylphenyl)isochinolin-4-carbonsäureamid
N-(2-(Diethylamino)ethyl)-6-hydroxy-1-(2-methylphenyl)isochinolin-4-carbonsäureamid
N-(2-(Diethylamino)ethyl)-6-methyl-1-(2-methylphenyl)isochinolin-4-carbonsäureamid
1-(2-Methylphenyl)isochinolin-4-carbonsäure, (3-(Diethylamino)propylester
N-(2-(Diethylamino)ethyl)-1-(4-methoxyphenyl)isochinolin-4-carbonsäureamid
1-(2-Chlorphenyl)-N-(3-dimethylamino)propyl)isochinolin-4-carbonsäureamid
1-Phenylisochinolin-4-carbonsäure, (2-(Piperidin-1-yl)ethyl)ester
1-Phenylisochinolin-4-carbonsäure, (2-(Morpholin-4-yl)ethyl)ester
N-(2-(Diethylamino)ethyl)-N-ethyl-1-phenylisochinolin-4-carbonsäureamid
N-(2-(Diethylamino)ethyl)-N-ethyl-1-(4-methylphenyl)isochinolin-4-carbonsäureamid
N-(2-(Diethylamino)ethyl)-1-(2-methoxyphenyl)isochinolin-4-carbonsäureamid
1-(2-Hydroxyphenyl)isochinolin-4-carbonsäure, (3-(Diethylamino)propyl)ester
N-(2-(Diethylamino)ethyl)-6,7-dimethoxy-1-(2,4-dimethylphenyl)isochinolin-4-carbonsäureamid
6,7-Dimethyl-1-phenylisochinolin-4-carbonsäure, (3-(Dimethylamino)propyl)ester
N-(2-(Diethylamino)ethyl)-6,8-dimethyl-1-(2-methylphenyl)isochinolin-4-carbonsäureamid
N-(3-(Dimethylamino)-2-propyl)-1-(2-methylphenyl)isochinolin-4-carbonsäureamid
N-(2-(Dimethylamino)-3-propyl)-1-(2-methylphenyl)isochinolin-4-carbonsäureamid
1-(2-Chlorphenyl)-N-(3-(dimethylamino)-2-propyl)isochinolin-4-carbonsäureamid
6-Chlor-N-(2-(diethylamino)ethyl)-1-(2-methylphenyl)isochinolin-4-carbonsäureamid
7-Methoxy-1-phenylisochinolin-4-carbonsäure, (3-(Dimethylamino)propyl)ester
N-(2-(Diisopropylamino)ethyl)-1-(2-methylphenyl)isochinolin-4-carbonsäureamid
1-(4-Aminophenyl)-N-(2-(diethylamino)ethyl)isochinolin-4-carbonsäureamid
N-(3-(Diethylamino)propyl)-1-(4-hydroxyphenyl)isochinolin-4-carbonsäureamid
N-(2-(Dibutylamino)ethyl)-1-phenylisochinolin-4-carbonsäureamid
N-(2-(Ethylamino)ethyl)-1-phenylisochinolin-4-carbonsäureamid
1-Phenylisochinolin-4-carbonsäure, (3-(Ethylamino)propyl)ester
N-(3-(Ethylamino)-2-propyl)-1-(2-methylphenyl)isochinolin-4-carbonsäureamid
N-(2-(Diethylamino)ethyl)-7-methyl-1-(2-methylphenyl)isochinolin-4-carbonsäureamid
N-(2-(Diisopropylamino)-1-(2,3-dimethylphenyl)isochinolin-4-carbonsäureamid
7-Fluor-1-phenylisochinolin-4-carbonsäure, (3-(Diethylamino)propyl)ester

N-(3-(Diethylamino)-2-propyl)-6,7-dimethoxy-1-(2-methylphenyl)isochinolin-4-carbonsäureamid
1-(4-Chlorphenyl)-N-(2-(diisopropylamino)ethyl)isochinolin-4-carbonsäureamid
1-(4-Amino-2-methylphenyl)-N-(2-(diethylamino)ethyl)isochinolin-4-carbonsäureamid
N-(4-(Diethylamino)-2-butyl)-1-(2-methylphenyl)isochinolin-4-carbonsäureamid
1-(4-Hydroxyphenyl)-N-(2-(thiomorpholin-4-yl)ethyl)isochinolin-4-carbonsäureamid
N-(2-(Dipropylamino)ethyl)-1-(2-methylphenyl)isochinolin-4-carbonsäureamid
1-(2,3-Dimethylphenyl)isochinolin-4-carbonsäure, (2-(Dibutylamino)ethyl)ester
N-(2-(Diethylamino)ethyl)-1-(3-nitrophenyl)isochinolin-4-carbonsäureamid
N-(2-(Diethylamino)ethyl)-7-nitro-1-phenylisochinolin-4-carbonsäureamid
1-(2-Methylphenyl)-N-(2-(pyrrolidin-1-yl)ethyl)isochinolin-4-carbonsäureamid
1-(2,3-Dimethylphenyl)-N-(2-(4-methylpiperazin-1-yl)ethyl)isochinolin-4-carbonsäureamid
1-Phenylisochinolin-4-carbonsäure, (1-Methylpiperidin-3-yl)ester
N-((1-Ethylpyrrolidin-2-yl)methyl)-1-(4-methoxyphenyl)isochinolin-4-carbonsäureamid
1-(2-Methylphenyl)isochinolin-4-carbonsäure, (Piperidin-4-yl)ester
1-(2-Chlorphenyl)isochinolin-4-carbonsäure, ((1-Methylpyrrolidin-3-yl)methyl)ester
N-((1-Methylpyrrolidin-3-yl)methyl)-1-(2-fluorphenyl)isochinolin-4-carbonsäureamid
N-(1-Methylpiperidin-4-yl)-1-phenylisochinolin-4-carbonsäureamid
N-((1-Ethylpiperidin-2-yl)methyl)-1-(4-hydroxyphenyl)isochinolin-4-carbonsäureamid
6,7-Dimethyl-N-((1-ethylpyrrolidon-2-yl)methyl)-1-(2-methylphenyl)isochinolin-4-carbonsäureamid
6,7-Dimethyl-N-(1-ethylpiperidin-3-yl)-1-phenylisochinolin-4-carbonsäureamid
7-Chlor-N-(1-methylpiperidin-3-yl)-1-(2-methylphenyl)isochinolin-4-carbonsäureamid
6-Fluor-N-(piperidin-3-yl)-1-phenylisochinolin-4-carbonsäureamid
6,7-Dimethyl-1-phenylisochinolin-4-carbonsäure, (1-Ethylpyrrolidin-2-yl)methylester
N-((1-Isopropylpyrrolidin-2-yl)methyl)-1-(2-methylphenyl)isochinolin-4-carbonsäureamid
6-Methoxy-1-(4-nitrophenyl)isochinolin-4-carbonsäure, (3-Diethylamino-2-propyl)ester
1-(2-Chlor-6-fluorphenyl)isochinolin-4-carbonsäure, (2-(Diethylamino)ethyl)ester
N-(2-Diethylamino)ethyl)-1-(2-Chlor-6-fluorphenyl)isochinolin-4-carbonsäureamid
1-(2-Chlorphenyl)isochinolin-4-carbonsäure, (1-Ethylpiperidin-4-yl)ester
N-((2-Isopropylamino)ethyl)-6-methyl-1-(2-methylphenyl)isochinolin-4-carbonsäureamid
N-((1-Methylpyrrolidin-3-yl)-methyl-1-(2,3-dimethylphenyl)isochinolin-4-carbonsäureamid
N-(1-Isopropylpiperidin-4-yl)-1-(2-chlorphenyl)isochinolin-4-carbonsäureamid
1-(4-Aminophenyl)-N-((1-methylpiperidin-2-yl)methyl)isochinolin-4-carbonsäureamid
N-(2-(Diethylamino)ethyl)-1-(4-hydroxyphenyl)-6-methylisochinolin-4-carbonsäureamid
N-((1-Ethylpyrrolidin-2-yl)methyl)-1-(4-hydroxyphenyl)-6-methoxyisochinolin-4-carbonsäureamid

Die erfindungsgemäßen Verbindungen sind neue Verbindungen und haben wertvolle therapeutische Eigenschaften. So zeigen sie beispielsweise eine Wirkung auf das Herz-Kreislaufsystem. Sie heben besonders die durch Strophantin verursachten Arrhythmien beim Hund auf. Auf Grund dieser Eigenschaft können die erfindungsgemäßen Verbindungen als Wirkstoffe von antiarrhythmisch wirkenden Arzneimitteln angewandt werden.

Die pharmakologischen Versuche wurden wie folgt durchgeführt: Bei einem Hund beliebigen Geschlechts wird mit 35 mg/kg Nembutal, i. p. eine Narkose erzeugt. Der Blutdruck wird über Druckaufnehmer von der A. carotis aufgenommen und in üblicher Weise registriert. Das EKG wird mit Einstichelektroden von den Extremitäten abgeleitet. Die am wenigsten gestörte Ableitung (meistens die Ableitung II) wird sowohl auf einem Oscilloskop dargestellt als auch über einen Direktschreiber registriert. Die Herzfrequenz wird elektronisch aus den R-Zacken des EKG bestimmt und ebenfalls fortlaufend auf dem Direktschreiber als Kurve dargestellt. Bis zum Auftreten von ventrikulären Extrasystolen wird dem Hund jetzt K-Strophantin als intravenöse Dauerinfusion über eine Beinvene zugeführt (4 mcg/kg/ml und Minute). Bleiben die Störungen ohne weitere Strophantingabe über einen Zeitraum von etwa 5 Minuten bestehen, wird die Infusion beendet. Es wird jetzt geprüft, ob die Prüfsubstanz die Störungen zu beseitigen vermag. Als erfolgreich gewertet wird nur ein sofortiges Schwinden der Extrasystolen bei i. v. Gabe bzw. innerhalb von 15 Minuten bei i. d. Gabe. Die Substanz wird als 5 %ige Lösung in Dosen von 1 und 3 mg/kg verabreicht. Als wirksam bezeichnet wird ein Präparat, das in den angegebenen Dosen mindestens eine eindeutige Verminderung der Zahl der Extrasystolen hervorruft. Eine starke Wirkung liegt dann vor, wenn ein Präparat bei intravenöser Applikation für eine Dauer von etwa 20 Minuten die ventrikulären Extrasystolen völlig beseitigt.

Die Ergebnisse sind in Tabelle I zusammengefaßt.

(Siehe Tabelle 1 Seite 7 f.)

Die erfindungsgemäßen Verbindungen und ihre pharmakologisch verträglichen Salze sind als Antiarrhythmica innerhalb eines breiten Dosierungsbereiches wirksam. So können einem Erwachsenen 50-100 mg pro Tag intravenös injiziert oder 100-300 mg pro Tag peroral appliziert werden.

Die neuen Verbindungen können entweder allein oder mit physiologisch verträglichen Hilfs- oder Trägerstoffen vermischt angewandt werden. Für eine orale Anwendungsform werden die aktiven

Tabelle 1

| Verbindung gemäß Beispiel-Nr. | Formel | Antiarrhythmische Wirkung am Strophantin-vergifteten Hund (i.v.) | |
|---|---|---|---|
| | | Wirkstärke | Wirkdauer |
| 1 | 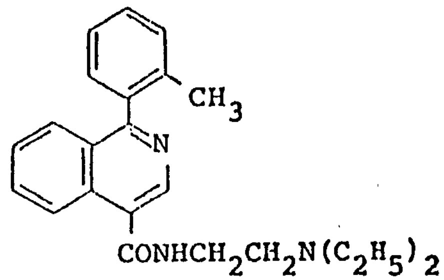 | sehr stark | sehr lang |
| 2 | 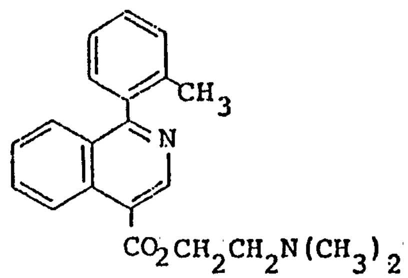 | stark | sehr lang |
| 3 | 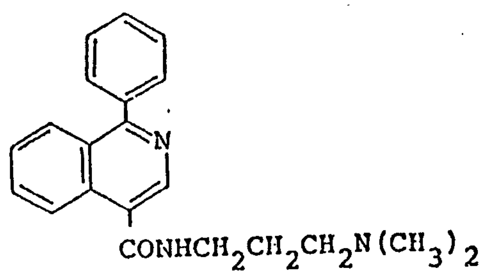 | stark | lang |

Verbindungen mit den dafür üblichen Substanzen vermischt und durch übliche Methoden in geeignete Darreichungsformen gebracht, wie Tabletten, Steckkapseln, wäßrige, alkoholische oder ölige Suspensionen oder wäßrige, alkoholische oder ölige Lösungen. Als inerte Träger können, z. B. Magnesiumcarbonat, Milchzucker oder Maisstärke verwendet werden. Dabei kann die Zubereitung sowohl als Trocken- oder Feuchtgranulat erfolgen. Als ölige Trägerstoffe oder Lösungsmittel kommen beispielsweise besonders pflanzliche und tierische Öle in Betracht wie z. B. Sonnenblumenöl oder Lebertran.

Eine besondere Anwendungsform liegt in der intravenösen Application. Zu diesem Zweck werden die aktiven Verbindungen oder deren physiologisch verträglichen Salze mit den dafür üblichen Substanzen in Lösung gebracht. Solche physiologisch verträglichen Salze werden z. B. mit folgenden Säuren gebildet : Chlor-, Brom- oder Jodwasserstoffsäure, Phosphorsäure, Schwefelsäure, Methylschwefelsäure, Amidosulfonsäure, Salpetersäure, Ameisensäure, Essigsäure, Propionsäure, Bernsteinsäure, Weinsäure, Milchsäure, Malonsäure, Fumarsäure, Oxalsäure, Zitronensäure, Äpfelsäure, Schleimsäure, Benzoesäure, Salicylsäure, Acetylaminoessigsäure, 4,4'-Methylen-bis-(3-hydroxy-2-naphthoesäure) (Embonsäure), Naphthalin-1,5-disulfonsäure, Ascorbinsäure, Phenylessigsäure, p-Amino-salicylsäure, Hydroxyäthansulfonsäure, Benzolsulfonsäure oder synthetische Harze, die saure Gruppen enthalten, z. B. solche mit Ionenaustauschwirkung. Als Lösungsmittel der entsprechenden physiologisch verträglichen Salze der aktiven Verbindungen für eine intravenöse Application kommen z. B. in Frage : Wasser, physiologische Kochsalzlösungen oder Alkohol, wie z. B. Äthanol, Propandiol oder Glycerin, daneben auch Zuckerlösungen wie z. B. Glukose- oder Mannitlösungen, oder auch eine Mischung aus den verschiedenen genannten Lösungsmitteln.

Beispiel 1

N-(2-(Diethylamino)ethyl)-1-(2-methylphenyl)isochinolin-4-carbonsäureamid

6,6 g 1-(2-Methylphenyl)isochinolin-4-carbonsäure werden in 100 ml Thionylchlorid 5 Stunden lang auf 50 °C erhitzt. Der Überschuß Thionylchlorid wird im Vakuum entfernt und der Rückstand mit Toluol aufgenommen und wieder im Vakuum eingeengt. Das so erhaltene Säurechlorid wird in 50 ml Chloroform gelöst und bei 0-10 °C zu 8,67 g 2-Diethylaminoethylamin in 140 ml Chloroform getropft. Die Reaktionsmischung bleibt über Nacht bei Raumtemperatur stehen, das Chloroform wird im Vakuum entfernt und der Rückstand zwischen Toluol und gesättigter Natriumhydrogencarbonatlösung verteilt. Die Toluolphase wird mit Magnesiumsulfat getrocknet und eingeengt. Das hellbraune Öl wird in wenig Äthanol gelöst und mit der äquimolaren Menge Chlorwasserstoff in Äthanol versetzt. Nach dem Einengen und lösen in Aceton kristallisiert 5,1 g als Hydrochlorid in fahlgelben Kristallen mit Schmelzpunkt 175-177 °C.

Das Ausgangsmaterial 1-(2-Methylphenyl)isochinolin-4-carbonsäure kann durch katalytische Enthalogenierung aus 3-Chlor-1-(2-methylphenyl)isochinolin-4-carbonsäure dargestellt werden :

40 g 3-Chlor-1-(2-methylphenyl)isochinolin-4-carbonsäure werden in 800 ml Methanol und 200 ml methanolischem Ammoniak gelöst und nach Zusatz von 2 g 10 %igem Palladium auf Thierkohle 6 Stunden bei 70 °C und 50 atm Wasserstoff hydriert. Die Lösung wird filtriert, eingeengt und der Rückstand in Wasser aufgenommen. Mit 2 n Salzsäure wird auf pH 2 eingestellt, und die Säure abfiltriert. Schmelzpunkt 254 °C.

Beispiel 2

1-(2-(Methylphenyl)isochinolin-4-carbonsäure, (2-Dimethylamino)ethylester

6,6 g 1-(2-Methylphenyl)isochinolin-4-carbonsäure werden mit 100 ml Thionylchlorid 5 Stunden bei 50 °C in das Säurechlorid überführt. Nach dem Entfernen des überschüssigen Thionylchlorids wird das Säurechlorid in 50 ml Chloroform gelöst und bei 0-10 °C zu 6,69 g 2-Dimethylaminoethanol in 140 ml Chloroform getropft. Nach 10 Stunden bei Raumtemperatur wird das Chloroform entfernt und der Rückstand in Toluol und gesättigter Natriumhydrogencarbonatlösung verteilt. Aus der Toluolphase isoliert man 6,0 g braunes Öl, das in äthanolischer Salzsäure 3,7 g das Hydrochlorid der gewünschten Verbindung mit Schmp. 176-179 °C ergibt.

Analog zu Beispiel 1 wurden folgende Verbindungen der Formel I hergestellt : (Tabelle II)

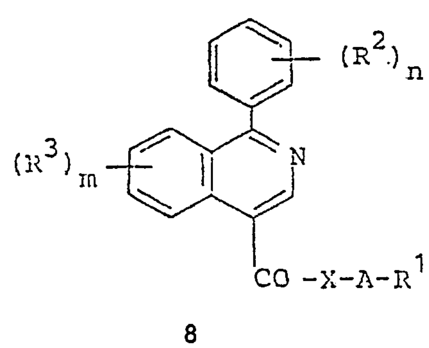

Tabelle 2

| Beispiel-Nr. | $-X-A-R^1$ | $(R^2)_n$ | $(R^3)_m$ | Salz/Schmp. °C |
|---|---|---|---|---|
| 3 | $NH-(CH_2)_3N(CH_3)_2$ | H | H | Oxalat / 125 - 126 |
| 4 | $NH(CH_2)_2-N(C_2H_5)_2$ | H | 6,7-di-$CH_3O$ | Dihydrochlorid / 162 - 164 |
| 5 | $NH-(CH_2)_3N(CH_3)_2$ | 2-$CH_3$ | H | Hydrochlorid / 205 - 207 |
| 6 | $NH-(CH_2)_2N(C_2H_5)_2$ | 2-$CH_3$ | 6,7-di-$CH_3O$ | Hydrochlorid / 100 - 102 |
| 7 | $NH-(CH_2)_3-N-(CH_3)_2$ | 2-$CH_3$ | 6,7-di-$CH_3O$ | Hydrochlorid / amorph |
| 8 | $NH-(CH_2)_3N(CH_3)_2$ | H | 6,7-di-$CH_3O$ | Dihydrochlorid/ 203 - 205 |
| 9 | $NH-(CH_2)_2N(C_2H_5)_2$ | H | 6-$CH_3$ | Hydrochlorid / 206 - 207 |
| 10 | $NH(CH_2)_2N(C_2H_5)_2$ | 4-$CH_3$ | H | Hydrochlorid / 176 - 178 |
| 11 | $NH(CH_2)_2N(C_2H_5)_2$ | 3-$NH_2$ | H | Hydrochlorid / amorph |
| 12 | $N(C_2H_5)-(CH_2)_2N(C_2H_5)_2$ | 2-$CH_3$ | H | Hydrochlorid / amorph |

Tabelle 2 (Fortsetzung)

| Beispiel-Nr. | X-A-$R^1$ | $(R^2)_n$ | $(R^3)_m$ | Salz/Schmp. °C |
|---|---|---|---|---|
| 13 | $O-CH(CH_3)-CH_2-N(C_2H_5)_2$ | 2-$CH_3$ | H | Hydrochlorid / 166 - 168 |
| 14 | $O-CH_2CH_2-N\left(CH(CH_3)_2\right)_2$ | 2-$CH_3$ | H | Hydrochlorid / 199 - 201 |
| 15 | 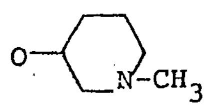 | 2-$CH_3$ | H | Oxalat / 170 - 173 |
| 16 | $NH\ (CH_2)_3N(C_3H_7)_2$ | 2-$CH_3$ | H | Hydrochlorid / 200 - 201 |
| 17 | $NH(CH_2)_4N(C_2H_5)_2$ | 2-$CH_3$ | H | Hydrochlorid / 168 - 170 |

## Beispiel 18

N-(2-(Diethylamino)ethyl)-1-phenylisochinolin-4-carbonsäureamid

5,2 g 3-Chlor-N-(2-(diethylamino)ethyl)-1-phenylisochinolin-4-carbonsäureamid werden in 225 ml Methanol und 30 ml methanolischem Ammoniak suspendiert und nach Zugabe von 1 g 10 %igen Palladium auf Tierkohle bei 40 °C und 1 atm. Wasserstoff hydriert. Nach Aufnahme der berechneten Menge Wasserstoff wird der Katalysator abfiltriert, das Filtrat eingeengt und zwischen Toluol und wässriger Kaliumcarbonatlösung verteilt. Die Toluolphase wird mit Wasser gewaschen, getrocknet, filtriert und eingeengt. Die zurückbleibenden 3,8 g hellgelben Öls werden in Isopropanol gelöst, und mit der äquimolaren Menge Oxalsäure in Isopropanol wird das Oxalat gefällt. Es entstehen 4,6 g mit Schmelzpunkt 115-118 unter Zersetzung.

Das Ausgangsmaterial 3-Chlor-N-(2-(diethylamino)ethyl)-1-phenyl-isochinolin-4-carbonsäureamid wird auf folgendem Weg dargestellt.

42,5 g 3-Chlor-1-phenylisochinolin-4-carbonsäure werden in 300 ml Thionylchlorid 4 Stunden auf dem Dampfbad erhitzt. Das überschüßige Thionylchlorid wird im Vakuum entfernt, der Rückstand mit Toluol versetzt und wieder im Vakuum eingeengt. 11,3 g des entstandenen Säurechlorids in 75 ml Chloroform werden bei 20 °C zu 13,1 g Diethylaminoethylamin in 100 ml Chloroform getropft. Nach 20 Stündigem Stehen bei Raumtemperatur wird zwischen Toluol und Wasser verteilt. Aus der Toluolphase werden 14 g eines braunen Öls isoliert, das in äthanolischer Oxalsäure ein kristallines Oxalat mit Schmelzpunkt 167-169 °C liefert.

## Beispiel 19

N-(2-(Diethylamino)ethyl)-1-phenylisochinolin-4-carbonsäureamid

5,2 g 1-Phenylisochinolin-4-carbonsäure werden mit 100 ml Thionylchlorid in das Säurechlorid überführt. Das rohe Säurechlorid wird in 20 ml Chloroform gelöst und bei 0-10 °C zu 8,6 g Diethylaminoethylamin in 100 ml Chloroform getropft. Die Reaktionslösung wird 10 Stunden bei Raumtemperatur gerührt, das Lösungsmittel im Vakuum entfernt und der Rückstand zwischen Toluol und Wasser verteilt. Aus der Toluollösung werden 4,1 g hellgelben Öls isoliert, das mit der äquimolaren Menge Oxalsäure in Isopropanol das kristalline Oxalat mit Schmp. 115-118° unter Zersetzung liefert.

Das Ausgangsmaterial 1-Phenylisochinolin-4-carbonsäure wird auf folgendem Weg dargestellt.

20,5 g 1-Phenylisochinolin werden in 100 ml Tetrachlorkohlenstoff gelöst und bei Raumtemperatur 16 g Brom zugetropft. Die Temperatur wird langsam gesteigert und die Mischung 1 Stunde am Rückfluß gehalten. In die siedende Reaktionsmischung tropft man während 2 Stunden 7,9 g Pyridin in 10 ml Tetrachlorkohlenstoff zu und erhitzt anschließend noch 18 Stunden zum Rückfluß. Die Reaktionslösung wird vom harzigen Niederschlag abdekantiert, eingeengt und der Rückstand aus Diisopropylether kristallisiert. 11,7 g 4-Brom-1-phenylisochinolin mit Schmp. 123-125 °C werden erhalten. 0,7 g 4-Brom-1-phenylisochinolin, 0,45 g Kupfercyanid und 10 ml Dimethylformamid werden 6 Stunden auf 160 °C erhitzt. Die Reaktionslösung wird abdekantiert, mit Methylenchlorid verdünnt und mit Wasser gewaschen. Aus der Methylenchloridphase werden 0,75 g Rohprodukt isoliert, aus dem durch Säulenchromatographie an Silicagel mit Chloroform/Essigester (98 : 2)-Gemisch 0,2 g 4-Cyano-1-phenylisochinolin mit Schmp. 138-140° erhalten werden. 0,2 g 4-Cyano-1-phenylisochinolin wird mit 10 ml 20 %iger Natriumhydroxidlösung 4 Stunden auf 100 °C erhitzt. Die Lösung wird abgekühlt und mit Essigsäure angesäuert. Der Niederschlag wird filtriert und getrocknet. Es werden 0,18 g 1-Phenyl-isochinolin-4-carbonsäure mit Schmp. 215-217° isoliert.

**Patentansprüche** (für die Vertragsstaaten : BE, CH, DE, FR, GB, IT, LI, LU, NL, SE)

1. Isochinolin-4-carbonsäurederivate der Formel I

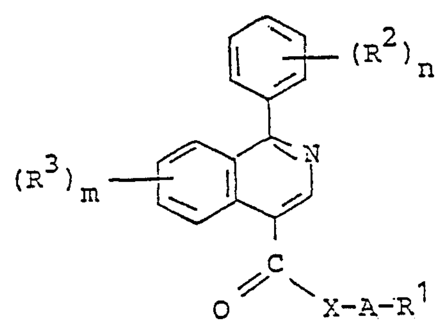

(I)

worin

m und n eins oder zwei,

X Sauerstoff oder Stickstoff, der mit Wasserstoff oder $C_1$-$C_6$-Alkyl substituiert ist,

A eine Bindung oder eine geradkettige oder verzweigte $C_1$-$C_8$-Alkylenkette,

$R^1$ entweder eine Aminogruppe der Formel

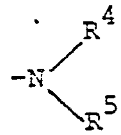

worin $R^4$ und $R^5$ gleich oder verschieden sind und Wasserstoff oder geradkettige oder verzweigte $C_1$-$C_8$-Alkylreste bedeuten oder

$R^1$ einen 3- bis 8-gliedrigen ein Stickstoffatom enthaltenden, entweder über ein Kohlenstoffatom oder ein Stickstoffatom mit A verbundenen Ring, worin das Stickstoffatom gegebenenfalls mit Wasserstoff oder $C_1$-$C_8$-Alkyl substituiert ist, und worin eine —$CH_2$-Gruppe durch Sauerstoff, Schwefel oder die —NH— oder N—$C_1$-$C_8$-Alkylgruppe ersetzt sein kann, und mit der Maßgabe, daß die Heteroatome in der Seitenkette (—X—A—$R^1$) durch mindestens 2 Kohlenstoffatome getrennt sind,

$R^2$ Wasserstoff, Halogen, Hydroxy, Nitro, Amino, $C_1$-$C_6$-Alkyl- oder $C_1$-$C_6$-Alkoxyreste, und

$R^3$ Wasserstoff, Halogen, Hydroxy, Nitro, Amino, $C_1$-$C_6$-Alkyl- oder $C_1$-$C_6$-Alkoxyreste, Benzyloxy-Methylendioxy- oder Äthylendioxy-Gruppe bedeuten sowie deren physiologisch verträgliche Säureadditionssalze.

2. Isochinolin-4-carbonsäurederivate der im Anspruch 1 angegebenen Formel I, worin n eins, X Sauerstoff oder Stickstoff, der mit Wasserstoff substituiert ist, A eine geradkettige $C_2$ bis $C_3$-Alkylengruppe, $R^1$ eine Aminogruppe der Formel

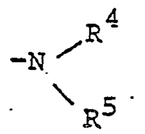

worin $R^4$ und $R^5$ gleich sind und $C_1$-$C_3$-Alkylreste darstellen, $R^2$ Wasserstoff oder $C_1$-$C_3$-Alkyl und $R^3$ Wasserstoff bedeuten, sowie deren physiologisch verträgliche Säureadditionssalze.

3. Verfahren zur Herstellung von Isochinolin-4-carbonsäurederivaten gemäß Anspruch 1, dadurch gekennzeichnet, daß man Verbindungen der Formel II

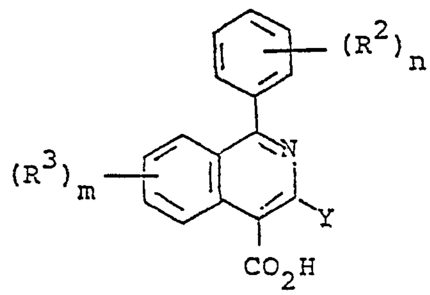

(II)

worin m, n, $R^2$ und $R^3$ die zu Formel I genannte Bedeutung haben und Y Wasserstoff oder Halogen bedeutet, mit einem Amin oder Alkohol der Formel HX—A—$R^1$, worin X, A und $R^1$ die obengenannte Bedeutung haben, umsetzt, eine erhaltene Verbindung, worin Y Halogen bedeutet, enthalogeniert, und die Reaktionsprodukte gegebenenfalls in die physiologisch verträglichen Säureadditionssalze überführt.

4. Pharmazeutisches Präparat enthaltend eine Verbindung gemäß Anspruch 1.

5. Verfahren zur Herstellung eines pharmazeutischen Präparates gemäß Anspruch 4, dadurch gekennzeichnet, daß man eine Verbindung gemäß Anspruch 1 in eine geeignete Darreichungsform überführt.

6. Verwendung von Verbindungen gemäß Anspruch 1 zur Herstellung eines Medikaments zur Behandlung von Arrhythmien.

**Patentansprüche** (für den Vertragsstaat AT)

1. Verfahren zur Herstellung von Isochinolin-4-carbonsäurederivaten der Formel I

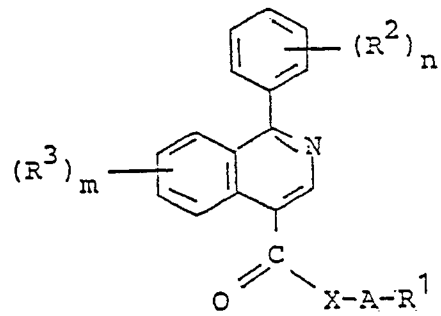

(I)

worin

m und n eins oder zwei,

X Sauerstoff oder Stickstoff, der mit Wasserstoff oder $C_1$-$C_6$-Alkyl substituiert ist,

A eine Bindung oder eine geradkettige oder verzweigte $C_1$-$C_8$-Alkylenkette,

$R^1$ entweder eine Aminogruppe der Formel

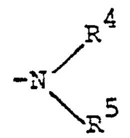

worin $R^4$ und $R^5$ gleich oder verschieden sind und Wasserstoff oder geradkettige oder verzweigte $C_1$-$C_8$-Alkylreste bedeuten oder

$R^1$ einen 3- bis 8-gliedrigen ein Stickstoffatom enthaltenden, entweder über ein Kohlenstoffatom oder ein Stickstoffatom mit A verbundenen Ring, worin das Stickstoffatom gegebenenfalls mit Wasserstoff oder $C_1$-$C_8$-Alkyl substituiert ist, und worin eine —$CH_2$-Gruppe durch Sauerstoff, Schwefel oder die —NH— oder N—$C_1$-$C_8$-Alkylgruppe ersetzt sein kann, und mit der Maßgabe, daß die Heteroatome in der Seitenkette (—X—A—$R^1$) durch mindestens 2 Kohlenstoffatome getrennt sind,

$R^2$ Wasserstoff, Halogen, Hydroxy, Nitro, Amino, $C_1$-$C_6$-Alkyl- oder $C_1$-$C_6$-Alkoxyreste, und

$R^3$ Wasserstoff, Halogen, Hydroxy, Nitro, Amino, $C_1$-$C_6$-Alkyl- oder $C_1$-$C_6$-Alkoxyreste, Benzyloxy-Methylendioxy- oder Äthylendioxy-Gruppe bedeuten sowie von deren physiologisch verträglichen Säureadditionssalzen, dadurch gekennzeichnet, daß man Verbindungen der Formel II

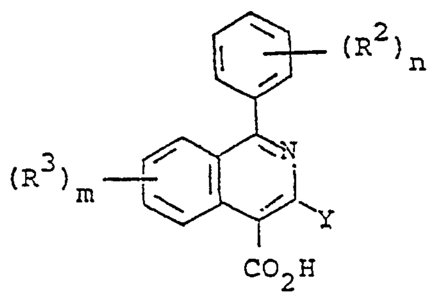

(II)

worin m, n, $R^2$ und $R^3$ die zu Formel I genannte Bedeutung haben und Y Wasserstoff oder Halogen bedeutet, mit einem Amin oder Alkohol der Formel HX—A—$R^1$, worin X, A und $R^1$ die obengenannte Bedeutung haben, umsetzt, eine erhaltene Verbindung, worin Y Halogen bedeutet, enthalogeniert und die Reaktionsprodukte gegebenenfalls in die physiologisch verträglichen Säureadditionssalze überführt.

2. Verfahren gemäß Anspruch 1, dadurch gekennzeichnet, daß man unter Verwendung entsprechender Ausgangsverbindungen Isochinolin-4-carbonsäurederivate der im Anspruch 1 angegebenen Formel I, worin n eins, X Sauerstoff oder Stickstoff, der mit Wasserstoff substituiert ist, A eine geradkettige $C_2$ bis $C_3$-Alkylengruppe, $R^1$ eine Aminogruppe der Formel

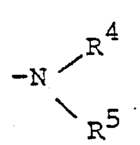

worin $R^4$ und $R^5$ gleich sind und $C_1$-$C_3$-Alkylreste darstellen, $R^2$ Wasserstoff oder $C_1$-$C_3$-Alkyl und $R^3$ Wasserstoff bedeuten, sowie deren physiologisch verträgliche Säureadditionssalze herstellt.

**Claims** (for the Contracting States : BE, CH, DE, FR, GB, IT, LI, LU, NL, SE)

1. An isoquinoline-4-carboxylic acid derivative of the formula I

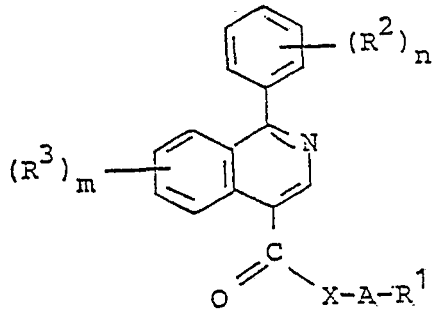

(I)

in which
m and n denote one or two,
X denotes oxygen or nitrogen which is substituted by hydrogen or $C_1$-$C_6$-alkyl,
A denotes a bond or a linear or branched $C_1$-$C_8$-alkylene chain,
$R^1$ either denotes an amino group of the formula

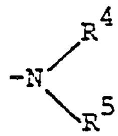

in which $R^4$ and $R^5$ are identical or different and denote hydrogen or linear or branched $C_1$-$C_8$-alkyl radicals, or

$R^1$ denotes a 3-membered to 8-membered ring which contains a nitrogen atom which ring is linked to A either *via* a carbon atom or *via* a nitrogen atom, and in which the nitrogen atom is optionally substituted by hydrogen or $C_1$-$C_8$-alkyl and in which one $-CH_2-$ group can be replaced by oxygen, sulfur or the —NH— or —N—$C_1$-$C_8$-alkyl group, subject to the proviso that the hetero-atoms in the side chain (—X—A—$R^1$) are separated by at least 2 carbon atoms,

$R^2$ denotes hydrogen, halogen, hydroxyl, nitro, amino or $C_1$-$C_6$-alkyl or $C_1$-$C_6$-alkoxy radicals and

$R^3$ denotes hydrogen, halogen, hydroxyl, nitro, amino, $C_1$-$C_6$-alkyl or $C_1$-$C_6$-alkoxy radicals or a benzyloxy, methylenedioxy or ethylenedioxy group, and physiologically acceptable acid addition salts thereof.

2. An isoquinoline-4-carboxylic acid derivative of the formula I indicated in claim 1, in which n denotes one, X denotes oxygen or nitrogen which is substituted by hydrogen, A denotes a linear $C_2$ to $C_3$ alkylene group, $R^1$ denotes an amino group of the formula

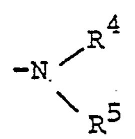

in which $R^4$ and $R^5$ are identical and represent $C_1$-$C_3$-alkyl radicals, $R^2$ denotes hydrogen or $C_1$-$C_3$-alkyl and $R^3$ denotes hydrogen, and physiologically acceptable acid addition salts thereof.

3. A process for the preparation of isoquinoline-4-carboxylic acid derivatives as claimed in claim 1, which comprises reacting compounds of the formula II

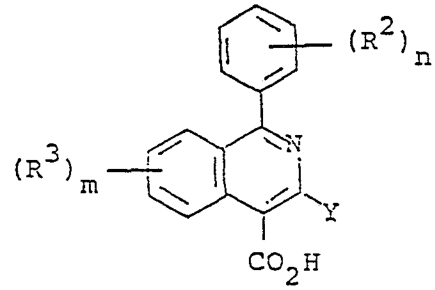

(II)

in which m, n, $R^2$ and $R^3$ have the meaning mentioned for formula I and Y denotes hydrogen or halogen, with an amine or alcohol of the formula HX—A—$R^1$ in which X, A and $R^1$ have the abovementioned meaning, dehalogenating the resulting compound in which Y denotes halogen, and, if desired, converting the reaction products into their physiologically acceptable acid addition salts.

4. A pharmaceutical formulation containing a compound as claimed in claim 1.

5. A process for the preparation of a pharmaceutical formulation as claimed in claim 4, which comprises converting a compound as claimed in claim 1 into a suitable form for administration.

6. The use of compounds as claimed in claim 1 in the treatment of arrythmias.

**Claims** (for the Contracting State AT)

1. A process for the production of isoquinoline-4-carboxylic acid derivatives of the formula I

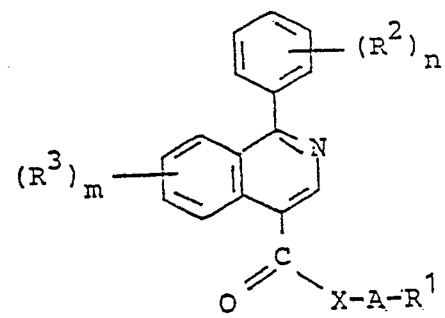

(I)

in which

m and n denote one or two,

X denotes oxygen or nitrogen which is substituted by hydrogen or $C_1$-$C_6$-alkyl,

A denotes a bond or a linear or branched $C_1$-$C_8$-alkylene chain,

$R^1$ either denotes an amino group of the formula

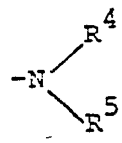

in which $R^4$ and $R^5$ are identical or different and denote hydrogen or linear or branched $C_1$-$C_8$-alkyl radicals, or

$R^1$ denotes a 3-membered to 8-membered ring which contains a nitrogen atom which ring is linked to A either *via* a carbon atom or *via* a nitrogen atom and in which the nitrogen atom is optionally substituted by hydrogen or $C_1$-$C_8$-alkyl and in which one —$CH_2$— group can be replaced by oxygen, sulfur or the —NH— or —N—$C_1$-$C_8$-alkyl group, subject to the proviso that the hetero-atoms in the side chain (—X—A—$R^1$) are separated by at least 2 carbon atoms,

$R^2$ denotes hydrogen, halogen, hydroxyl, nitro, amino or $C_1$-$C_6$-alkyl or $C_1$-$C_6$-alkoxy radicals and

$R^3$ denotes hydrogen, halogen, hydroxyl, nitro, amino, $C_1$-$C_6$-alkyl or $C_1$-$C_6$-alkoxy radicals or a benzyloxy, methylenedioxy or ethylenedioxy group, and physiologically acceptable acid addition salts thereof characterized in that compounds of the formula II

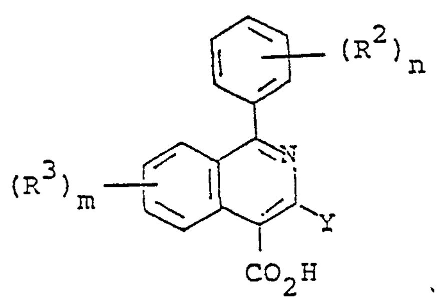

(II)

in which m, n, $R^2$ and $R^3$ have the meaning mentioned for formula I and Y denotes hydrogen or halogen, are reacted with an amine or alcohol of the formula HX—A—$R^1$ in which X, A and $R^1$ have the abovementioned meaning, that the resulting compounds in which Y denotes halogen are dehalogenated, and, if desired, the reaction products are converted into their physiologically acceptable acid addition salts.

2. A process according to claim 1, characterized in that an isoquinoline-4-carboxilic acid derivative of the formula I indicated in claim 1, in which n denotes one, X denotes oxygen or nitrogen which is substituted by hydrogen, A denotes a linear $C_2$ to $C_3$ alkylene group, $R^1$ denotes an amino group of the formula

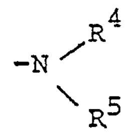

in which $R^4$ and $R^5$ are identical and represent $C_1$-$C_3$-alkyl radicals, $R^2$ denotes hydrogen or $C_1$-$C_3$-alkyl and $R^3$ denotes hydrogen, and physiologically acceptable acid addition salts thereof is produced by using the respective starting materials.

**Revendications** (pour les Etats contractants : BE, CH, DE, FR, GB, IT, LI, LU, NL, SE)

1. Dérivés de l'acide isoquinoléine-4-carboxylique de formule I

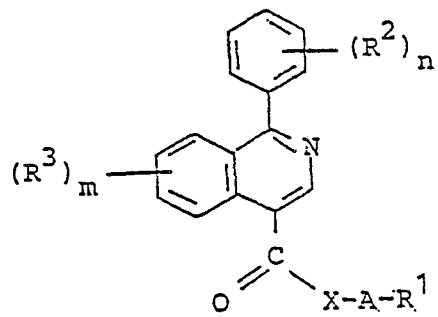

(I)

dans laquelle :

m et n sont 1 ou 2,

X représente l'oxygène ou l'azote, substitué par l'hydrogène ou un groupe alkyle en $C_1$-$C_6$,

A représente une liaison ou une chaîne alkylène en $C_1$-$C_8$, droite ou ramifiée,

$R^1$ représente un groupe amino de formule

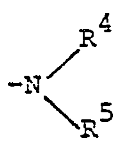

dans lequel $R^4$ et $R^5$ sont identiques ou différents et représentent l'hydrogène ou un groupe alkyle en $C_1$-$C_8$ à chaîne droite ou ramifiée, ou

$R^1$ représente un noyau de 3 à 8 chaînons, contenant un atome d'azote, relié à A par un atome de carbone ou un atome d'azote, l'atome d'azote étant éventuellement substitué par l'hydrogène ou un groupe alkyle en $C_1$-$C_8$, et un groupe —$CH_2$— pouvant être remplacé par l'oxygène, le soufre ou un groupe NH ou N-alkyle en $C_1$-$C_8$, à la condition que les hétéro-atomes dans la chaîne latérale (—X—A—$R^1$) soient séparés par au moins deux atomes de carbone,

$R^2$ représente l'hydrogène, un halogène, un groupe hydroxy, nitro, amino, alkyle en $C_1$-$C_6$ ou alcoxy en $C_1$-$C_6$, et

$R^3$ représente l'hydrogène, un halogène, un groupe hydroxy, nitro, amino, alkyle en $C_1$-$C_6$ ou alcoxy en $C_1$-$C_6$, benzyloxy, méthylènedioxy ou éthylènedioxy, ainsi que leurs sels d'addition avec des acides physiologiquement acceptables.

2. Dérivés de l'acide isoquinoléine-4-carboxylique de formule I selon la revendication 1, dans lesquels n est 1, X représente l'oxygène ou l'azote substitué par l'hydrogène, A représente un groupe alkylène en $C_2$-$C_3$ à chaîne droite, $R^1$ représente un groupe amino de formule

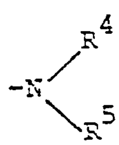

dans lequel $R^4$ et $R^5$ sont identiques et représentent un groupe alkyle en $C_1$-$C_3$, $R^2$ représente l'hydrogène ou un groupe alkyle en $C_1$-$C_3$ et $R^3$ représente l'hydrogène, ainsi que leurs sels d'addition avec des acides physiologiquement acceptables.

3. Procédé de préparation de dérivés de l'acide isoquinoléine-4-carboxylique selon la revendication 1, caractérisé en ce qu'on fait réagir des composés de formule II

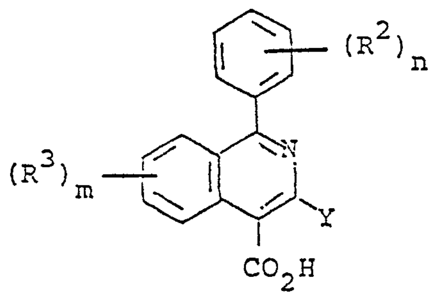

(II)

dans laquelle m, n, $R^2$ et $R^3$ ont les significations indiquées pour la formule I et Y représente l'hydrogène ou un halogène, avec une amine ou un alcool de formule HX—A—$R^1$, dans lequel X, A et $R^1$ ont les significations indiquées ci-dessus, on élimine l'halogène du composé obtenu dans lequel X représente un halogène, et on convertit éventuellement les produits de réaction en les sels d'addition avec des acides physiologiquement acceptables.

4. Composition pharmaceutique contenant un composé selon la revendication 1.

5. Procédé de préparation d'une composition pharmaceutique selon la revendication 4, caractérisé en ce qu'on convertit un composé selon la revendication 1 en une forme d'administration appropriée.

6. Utilisation de composés selon la revendication 1 pour la préparation d'un médicament pour le traitement des arythmies.

**Revendications** (pour l'Etat contractant AT)

1. Procédé de préparation de dérivés de l'acide isoquinoléine-4-carboxylique, de formule I

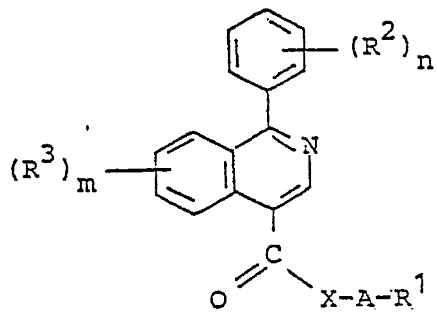

(I)

dans laquelle :

m et n sont 1 ou 2,

X représente l'oxygène ou l'azote, substitué par l'hydrogène ou un groupe alkyle en $C_1$-$C_6$,

A représente une liaison ou une chaîne alkylène en $C_1$-$C_8$, droite ou ramifiée,

$R^1$ représente un groupe amino de formule

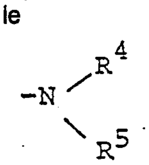

dans lequel $R^4$ et $R^5$ sont identiques ou différents et représentent l'hydrogène ou un groupe alkyle en $C_1$-$C_8$ à chaîne droite ou ramifiée, ou

$R^1$ représente un noyau de 3 à 8 chaînons contenant un atome d'azote, relié à A par un atome de carbone ou un atome d'azote, l'atome d'azote pouvant éventuellement être substitué par l'hydrogène ou un groupe alkyle en $C_1$-$C_8$, et un groupe —$CH_2$ pouvant être remplacé par l'oxygène, le soufre ou un groupe NH ou N-alkyle en $C_1$-$C_8$, et à la condition que les hétéro-atomes dans la chaîne latérale (—X—A—$R^1$) soient séparés par au moins 2 atomes de carbone,

$R^2$ représente l'hydrogène, un halogène, un groupe hydroxy, nitro, amino, alkyle en $C_1$-$C_6$ ou alcoxy en $C_1$-$C_6$, et

$R^3$ représente l'hydrogène, un halogène, un groupe hydroxy, nitro, amino, alkyle en $C_1$-$C_6$ ou alcoxy en $C_1$-$C_6$, benzyloxy, méthylènedioxy ou éthylènedioxy, ainsi que de leurs sels d'addition avec des acides physiologiquement acceptables, ce procédé étant caractérisé en ce qu'on fait réagir des composés de formule II

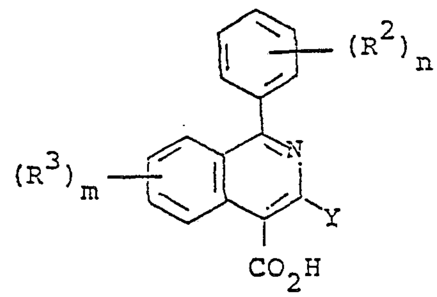

(II)

dans laquelle, m, n, $R^2$ et $R^3$ ont les significations indiquées pour la formule I et Y représente l'hydrogène ou un halogène, avec une amine ou un alcool de formule HX—A—$R^1$, dans lequel X, A et $R^1$ ont les significations indiquées ci-dessus, on élimine l'halogène du composé obtenu dans lequel Y est un halogène et éventuellement on convertit les produits de réaction en les sels d'addition avec des acides physiologiquement acceptables.

2. Procédé selon la revendication 1, caractérisé en ce que par utilisation des composés de départ correspondants, on prépare des dérivés de l'acide isoquinoléine-4-carboxylique de Formule I selon la revendication 1, dans lesquels n est 1, X représente l'oxygène ou l'azote, substitué par l'hydrogène, A représente un groupe alkylène en $C_2$-$C_3$ à chaîne droite, $R^1$ représente un groupe amino de formule

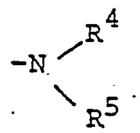

dans lequel $R^4$ et $R^5$ sont identiques et représentent un groupe alkyle en $C_1$-$C_3$, $R^2$ représente l'hydrogène ou un groupe alkyle en $C_1$-$C_3$ et $R^3$ représente l'hydrogène, ainsi que de leurs sels d'addition avec des acides physiologiquement acceptables.